# EUROPEAN PATENT APPLICATION

(11) **EP 2 704 439 A1**
(43) Date of publication of application: **05.03.2014**
(21) Application number: 13181839.5
(22) Date of filing: 27.08.2013
(51) Int. Cl.: H04N 19/00, G06F 19/00, A61B 1/00

(54) **Medical image recording apparatus, recording method of the same, and medical image recording program**

(30) Priority: 28.08.2012 JP 2012187657
(71) Applicant: Fujifilm Corporation, Minato-ku Tokyo (JP)
(72) Inventor: Miura, Nobuyuki, Tokyo (JP)
(74) Representative: Klunker . Schmitt-Nilson . Hirsch

(57) **Abstract**

A medical image recording apparatus includes: a storage unit that records, at a first compression rate, moving image data acquired by a modality apparatus; a control section that determines degrees of importance of individual frame images constituting the moving image data; and a compression processing section that, if a situation of the moving image data recorded in the storage unit satisfies a prescribed condition, (i) compresses the moving image data at a second compression rate that is smaller than the first compression rate and (ii) compresses each frame image of the moving image data at a compression rate that is smaller than the first compression rate and larger than the second compression rate and that decreases as the degree of importance decreases.

## Description

### BACKGROUND

### 1. Technical Field

The present invention relates to a medical image recording apparatus, a recording method of the same, and a medical image recording program. More particularly, the invention relates to a medical image recording apparatus etc. which are suitable for reduction of the amount of recording data to be recorded in a recording medium because the compression rate of each image data of medical image data is set according to its importance.

### 2. Related Art

In recent medical practices, diagnoses have come to be made on the basis of medical image data that are acquired with various modalities such as electronic endoscopes, ultrasonic diagnostic instruments, and radioscopic apparatus.

As for image data acquired by electronic endoscopes, in a typical case, image data that is taken upon manipulation of a release switch of an endoscope is recorded as still image data. Image data may also be recorded in the form of moving image data.

For example, in the image recording apparatus disclosed in Patent document 1 (JP-A-2005-066057), image data acquired by an electronic endoscope is always recorded as moving image data in, for example, an uncompressed manner. To facilitate later edit work, the moving image data is divided at proper positions by manipulations of a switch that is provided in the electronic endoscope.

In general, to record moving image data, recording media are required to have very large capacities. In view of this, in the image recording apparatus disclosed in Patent document 2 (JP-A-H02-263269), first, moving image data acquired by an electronic endoscope is recorded after being subjected to low-compression reversible compression processing. After a lapse of a prescribed period from the recording or the reading frequency has become lower than a prescribed value, the recorded moving image data is subjected to high-compression irreversible compression processing and the original reversibly compressed moving image data is erased, that is, the original reversibly compressed moving image data is replaced by the irreversibly compressed moving image data. In this manner, the data amount is reduced and the capacity of a recording medium is saved.

### SUMMARY OF INVENTION

In the image recording apparatus disclosed in Patent document 2, moving image data is subjected to uniform compression processing, in which case portions, relatively high in importance, of the moving image data may be made low in image quality. On the other hand, the required image quality depends on the examination/treatment type and the modality type. If the compression rate is set for a case that requires highest image quality, the data amount cannot be reduced effectively.

An object of the present invention is to reduce the data amount effectively while maintaining image quality levels that are suitable for uses in recording and storage of medical image data.
(1) According to an aspect of the invention, a medical image recording apparatus includes: a storage unit that records, at a first compression rate, moving image data acquired by a modality apparatus; a control section that determines degrees of importance of individual frame images constituting the moving image data; and a compression processing section that, if a situation of the moving image data recorded in the storage unit satisfies a prescribed condition, (i) compresses the moving image data at a second compression rate that is smaller than the first compression rate and (ii) compresses each frame image of the moving image data at a compression rate that is smaller than the first compression rate and larger than the second compression rate and that decreases as the degree of importance decreases.
(2) According to another aspect of the invention, a medical image recording method includes: compressing moving image data acquired by a modality apparatus at a first compression rate and recording resulting moving image data in a storage unit; determining degrees of importance of individual frame images constituting the moving image data recorded in the storage unit; and if a situation of the moving image data recorded in the storage unit satisfies a prescribed condition, (i) compressing the moving image data at a second compression rate that is smaller than the first compression rate and (ii) compressing each frame image at a compression rate that is smaller than the first compression rate and larger than the second compression rate and that decreases as the degree of importance decreases.
(3) According to another aspect invention, a medical image recording program causes a computer to execute: compressing moving image data acquired by a modality apparatus at a first compression rate and recording resulting moving image data in a storage unit; determining degrees of importance of individual frame images constituting the moving image data recorded in the storage unit; and if a situation of the moving image data recorded in the storage unit satisfies a prescribed condition, (i) compressing the moving image data at a second compression rate that is smaller than the first compression rate and (ii) compressing each frame image at a compression rate that is smaller than the first compression rate and larger than the second compression rate and that decreases as the degree of importance decreases.

The invention makes it possible to reduce the data amount effectively while maintaining image quality levels that are suitable for uses in recording and storage of medical image data.

### BRIEF DESCRIPTION OF DRAWINGS

Fig. 1 shows the configuration of an example electronic endoscope system according to an embodiment of the present invention.
Fig. 2 shows the structure of a tip portion of a scope of the electronic endoscope system of Fig. 1.
Fig. 3 is a functional block diagram of the electronic endoscope system of Fig. 1.
Fig. 4 illustrates a method for determining the importance of each frame image in the electronic endoscope system of Fig. 1.
Fig. 5 is a flowchart showing a procedure of compression processing which is performed on unprocessed data of moving image data stored in a hard disk drive (HDD) as shown in Fig. 3.
Fig. 6 shows example compression rate table data that is set in a compression rate setting section shown in Fig. 3.
Fig. 7 shows another example compression rate table data that is set in the compression rate setting section shown in Fig. 3.
Fig. 8 shows the configuration of an example intra-hospital system according to the embodiment.
Fig. 9 shows example compression rate table data that is set in a compression rate setting section of a server shown in Fig. 8.

### DESCRIPTION OF EMBODIMENTS

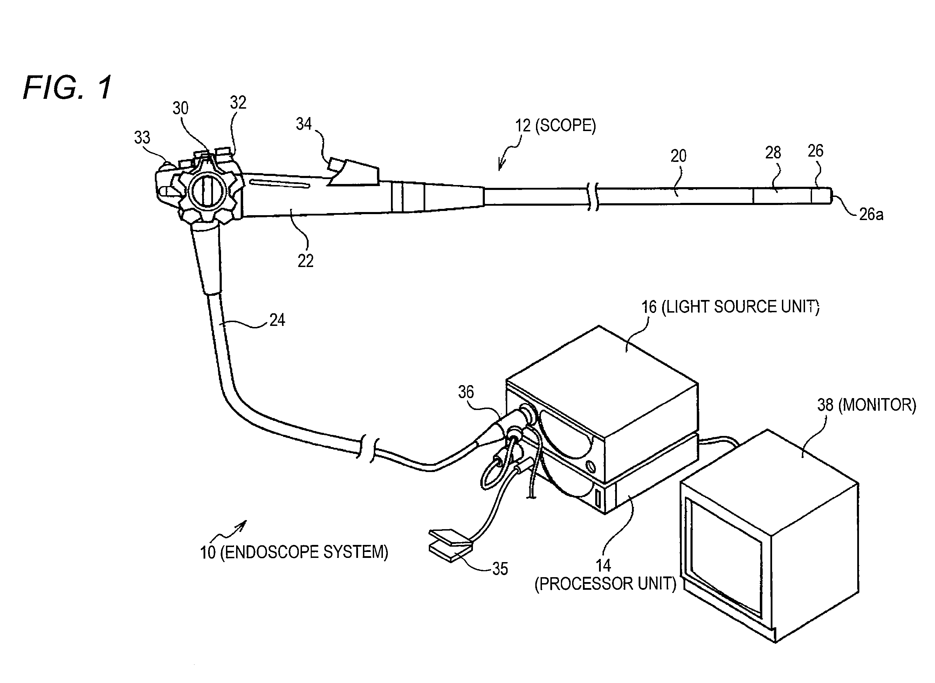

Fig. 1 shows the configuration of an example electronic endoscope system according to an embodiment of the present invention.

The electronic endoscope system 10 shown in Fig. 1 is constituted by a scope (endoscope) 12 and a processor unit 14 and a light source unit 16 which constitute a main body. In the embodiment, the processor unit 14 also functions as a medical image recording apparatus.

The scope 12 is equipped with a flexible insertion member 20 to be inserted into the body cavity of a patient (subject), a manipulation unit 22 which is continuous with a proximal portion of the insertion member 20, and a universal cord 24 which is connected to the processor unit 14 and the light source unit 16.

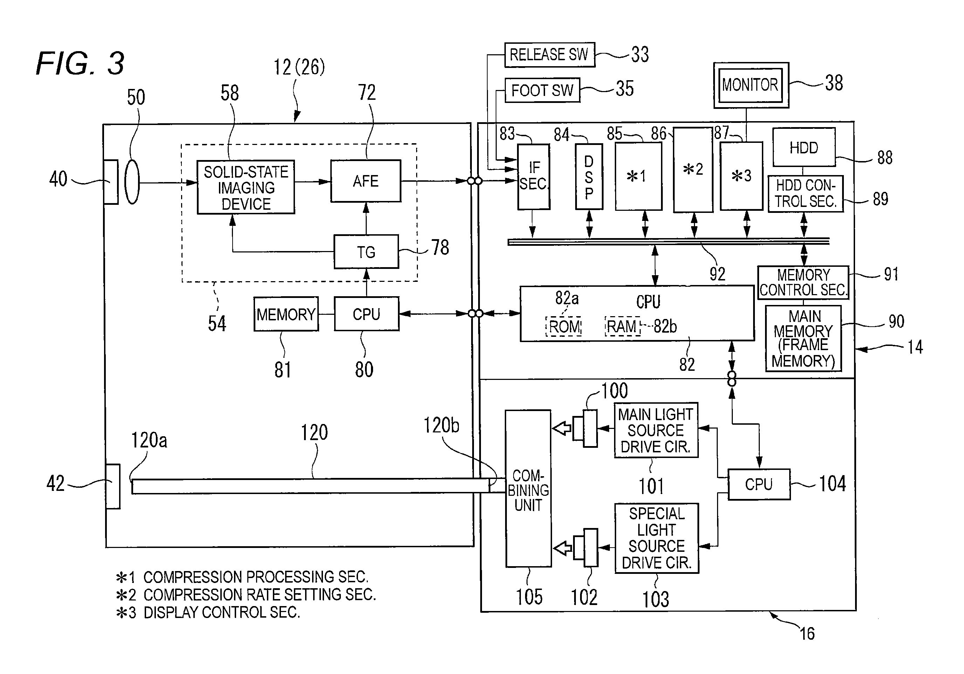

A tip portion 26 is continuous with the tip of the insertion member 20 and incorporates an imaging chip (imaging device) 54 (see Fig. 3) for body cavity shooting. A curvable portion 28 which is a connection of plural curvable pieces is disposed behind the tip portion 26. When an angle knob 30 which is provided in the manipulation unit 22 is manipulated, a wire that is inserted in the insertion member 20 is pushed or pulled and the curvable portion 28 is thereby curved in the top, bottom, left, or right direction. Thus, the tip portion 26 is directed to a desired direction in a body cavity.

A release switch 33 for the imaging device 54 is disposed near the angle knob 30. An endoscope operator pushes the release switch 33 when he or she wants to take a high-resolution still image.

A connector 36 is provided at the proximal end of the universal cord 24. Being of a composite type, the connector 36 is connected to not only the processor unit 14 but also the light source unit 16.

The processor unit 14 supplies power to the scope 12 and drive-controls the imaging device 54 via a cable that is inserted through the universal cord 24. The processor unit 14 receives an imaging signal that is transmitted from the imaging device 54 through the cable, and converts it into image data by subjecting it to various kinds of signal processing (image processing).

The image data thus produced by the processor unit 14 is displayed as an endoscope shot image (observation image) on a monitor 38 which is connected to the processor unit 14 by a cable. The processor unit 14 is also electrically connected to the light source unit 16 via the connector 36. The processor unit 14 thus controls the operations of the electronic endoscope system 10 (including the light source unit 16) in a unified manner.

A foot switch 35 is connected to the processor unit 14. Placed near a foot of an endoscope operator, the foot switch 35 supplies an on signal (push-down signal) or an off signal (release signal) to the processor unit 14.

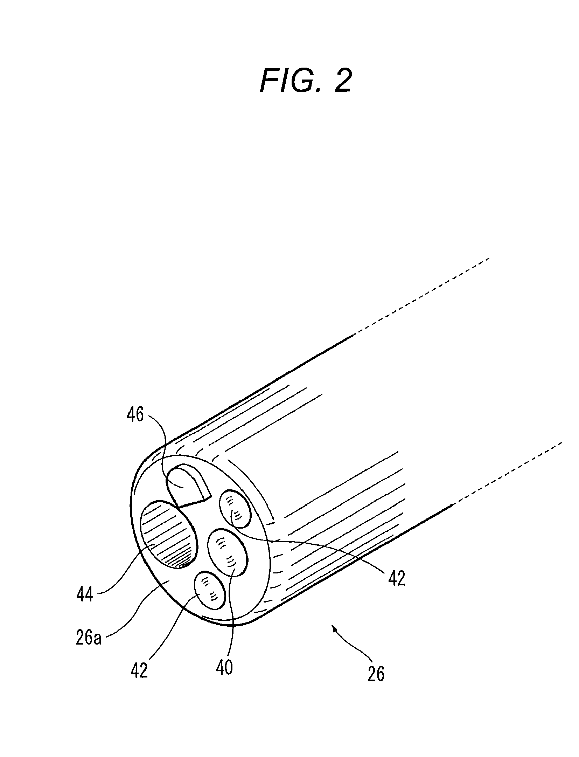

Fig. 2 shows the structure of the tip portion 26 of the scope 12.

A tip face 26a of the tip portion 26 is provided with an observation window 40, illumination windows 42, a forceps outlet 44, and an air/water feed nozzle 46.

The observation window 40 is disposed near the center of the tip face 26a and is deviated to one side. The two illumination windows 42 are disposed symmetrically with respect to the observation window 40, and illuminate an observation part in a body cavity with illumination light beams coming from the light source unit 16.

The forceps outlet 44 is connected to a forceps pipe (not shown) which is disposed in the insertion member 20, and is continuous with a forceps inlet 34 (see Fig. 1) which is provided in the manipulation unit 22. Any of various treatment tools whose tips are provided with an injection needle, a high-frequency scalpel, etc. is inserted through the forceps inlet 34 and the tip of the inserted treatment tool is put into the body cavity through the forceps outlet 44.

The air/water feed nozzle 46 jets out cleaning water or air that is supplied from an air/water feeding device incorporated in the light source unit 16 toward the observation window 40 or into the body cavity in response to a manipulation on an air/water feed button 32 (see Fig. 1) provided in the manipulation unit 22.

Fig. 3 is a functional block diagram of a control system of the electronic endoscope system 10.

As shown in Fig. 3, the scope 12 is equipped with a solid-state imaging device (image sensor) 58, an analog signal processing circuit (analog front end (AFE)) 72, a timing generator (TG) 78, and a CPU 80.

A memory 81 such as an EEPROM is connected to the CPU 80. Data specific to the scope 12 (e.g., its ID number) and data specific to the solid-state imaging device 58 are stored in the memory 81.

The TG 78 generates drive pulses (vertical and horizontal scanning pulses, a reset pulse, etc.) for the solid-state imaging device 58 and sync pulses for the AFE 72 under the control of the CPU 80 which communicates with a CPU 82 of the processor unit 14. Driven by the drive pulses supplied from the TG 78, the solid-state imaging device 58 photoelectrically converts an optical image formed on the imaging surface by an objective optical system 50 into an image signal and outputs it as moving image data having a prescribed frame rate (e.g., 30 frames/sec).

The AFE 72 is constituted by a correlated double sampling (CDS) circuit, an automatic gain control (AGC) circuit, and an A/D converter. The CDS circuit performs correlated double sampling processing on an image signal that is output from the solid-state imaging device 58 and thereby eliminates reset noise and amplifier noise that occur in the solid-state imaging device 58.

The AGC circuit amplifies the image signal from which noise has been eliminated by the CDS circuit at a gain (amplification factor) that is specified by the CPU 80. The A/D converter converts the image signal that has been amplified by the AGC circuit into a digital signal having a prescribed number of bits and outputs the latter. The output image signal (digital image signal) that has been digitized by the AFE 72 is input to the processor unit 14 via a signal line.

The processor unit 14 is equipped with the CPU 82, an interface section 83, an image processing circuit (DSP) 84, a compression processing section 85, a compression rate setting section 86, a display control section 87, an HDD control section 89 which is connected to a hard disk drive (HDD) 88 (example large-capacity storage medium), a memory control section 91 which is connected to a main memory (frame memory) 90, and a bus 92 which connects the above components to each other.

The monitor 38 (see Fig. 1) is connected to the display control section 87. A digital image signal that is output from the AFE 72, a signal of the release switch 33, and an on/off signal of the foot switch 35 are input to the interface section 83.

The CPU 82 controls the individual sections of the processor unit 14, and controls the whole of the electronic endoscope system 10 in a unified manner. A ROM 82a which is incorporated in the CPU 82 is stored in advance with various programs, control data, etc. for controlling the operations of the processor unit 14. A program being run by the CPU 82, related data, etc. are stored temporarily in a RAM 82b which is incorporated in the CPU 82.

Instructed by the CPU 82, the DSP 84 generates image data by performing various kinds of image processing such as color interpolation, color separation, color balance adjustment, gamma correction, and image emphasis processing on an image signal that is input from the AFE 72. The image data that has been subjected to the image processing in the DSP 84 and then developed in the main memory 90 is displayed on the monitor 38 via the display control section 87.

The light source unit 16 is constituted by a main light source 100, a main light source drive circuit 101, a special light source 102, a special light source drive circuit 103, a CPU 104, and a combining unit 105. The CPU 104 communicates with the CPU 82 of the processor unit 14 and controls the main light source drive circuit 101 and the special light source drive circuit 103.

The main light source 100 emits white light, and the special light source 102 emits special light in a narrow band centered at 420 nm, for example. The white light or special light passes through the combining unit 105 and shines on an incident end 120b of a light guide 120.

To observe a body cavity using the above-configured electronic endoscope system 10, the scope 12, the processor unit 14, the light source unit 16, and the monitor 38 are powered on and the insertion member 20 of the scope 12 is inserted into the body cavity. A moving image of a part in the body cavity that is taken by the solid-state imaging device 58 while that part is illuminated by illumination light coming from the light source unit 16 is displayed on the monitor 38 and observed.

Image data taken by the solid-state imaging device 58 is input to the interface section 83 as moving image data having a prescribed frame rate. The DSP 84 performs the above-mentioned various kinds of image processing while temporarily storing the image data in the main memory 90 frame by frame. The display control section 87 displays the image data on the monitor 38.

The electronic endoscope system 10 always records moving image data taken by the solid-state imaging device 58; the electronic endoscope system 10 stores, in the HDD 88, all of moving image data that is obtained from a start of insertion of the insertion member 20 etc. of the scope 12 into a subject to an end of their removal. That is, frame image data that are stored in the main memory 90 and subjected to image processing one after another are transferred to the HDD 88 and stored temporarily there.

In the electronic endoscope system 10, moving image data taken by the solid-state imaging device 58 is temporarily stored in the HDD 88 without being compressed, that is, with the compression rate (the ratio the amount of compressed data to the amount of original data) set at 100% (first compression rate). Alternatively, moving image data taken by the solid-state imaging device 58 may be temporarily stored in the HDD 88 after being transferred to and compressed by the compression processing section 85.

When a large amount of moving image data has been stored in the HDD 88, the residual capacity of the HDD 88 becomes small. In view of this, in the electronic endoscope system 10, moving image data that satisfies a prescribed condition is compressed to increase the residual capacity of the HDD 88. Furthermore, in the electronic endoscope system 10, important ones of the frame images constituting moving image data to be compressed are compressed at compression rates corresponding to their degrees of importance and resulting frame images are stored in the HDD 88.

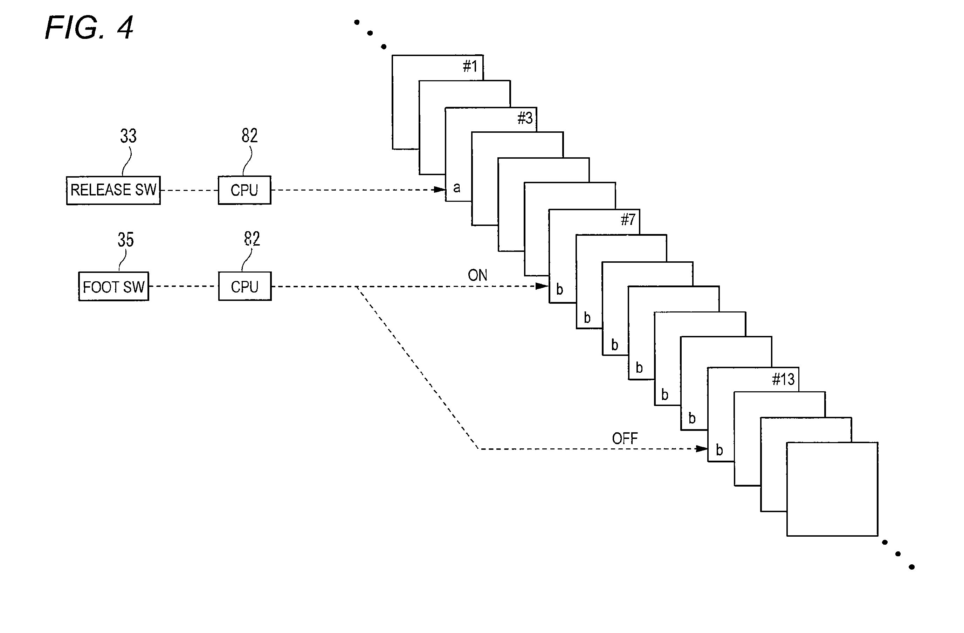

Fig. 4 illustrates a method for determining the importance of each frame image.

Consecutive frame images of moving image data that continues to be output from the solid-state imaging device 58 are developed in the main memory 90 in order. The DSP 84 performs image processing on the individual images and the display control section 87 displays them on the monitor 38.

Frame images as subjected to image processing are transferred from the memory control section 91 to the HDD control section 89 and stored in the HDD 88 with such timing that is somewhat delayed from the monitor display timing. The frame images thus stored in the HDD 88 are deleted from the main memory 90 by the memory control section 91, and a resulting free space of the main memory 90 for temporal storage of frame images subsequently output from the solid-state imaging device 58 and as a work area. In this manner, frame image data are transferred sequentially from the solid-state imaging device 58 to the main memory 90 and displayed on the monitor 38 as observation images.

If the endoscope operator finds an image to pay attention to during observation of monitoring images and pushes the release switch 33 (see Fig. 1) of the scope 12, a resulting release signal is taken from the interface section 83. When receiving the release signal, the CPU 82 attaches, to the frame image data (in the example of Fig. 4, #3 data) stored in the main memory 90 as corresponding to the frame image that is displayed on the monitor 38 at the time of the push of the release switch 33, an importance tag "a" as additional information (tag information) which indicates that this frame image is more important than other ones.

If the endoscope operator determines during observation of images displayed on the monitor 38 that a partial moving image of the part being observed should be recorded, he or she steps on the foot switch 35 and then raises his or her foot when determining that the recording of the partial moving image should be finished. As a result, a foot switch 35 push-down timing signal (moving image recording on signal) and a release timing signal (moving image recording off signal) are taken from the interface section 83. The CPU 82 attaches, to the frame image data (in the example of Fig. 4, #7 data) stored in the main memory 90 as corresponding to the frame image that is displayed on the monitor 38 at the time of the start of the partial moving image recording, an importance tag "b" as additional information which indicates that this frame image is more important than other ones. The CPU 82 attaches importance tags "b" to the succeeding frame image data, the last one being the frame image data (in the example of Fig. 4, #13 data) stored in the main memory 90 as corresponding to the frame image that is displayed at the time of the end of the partial moving image recording.

As a result of the above operation, as moving image data taken by the solid-state imaging device 58 are temporarily stored in the HDD 88, frame image data to which the importance tags "a" and "b" are attached are transferred to the HDD 88 and stored there. The importance tags "a" and "b" may indicate either the same degree of importance or different degrees of importance.

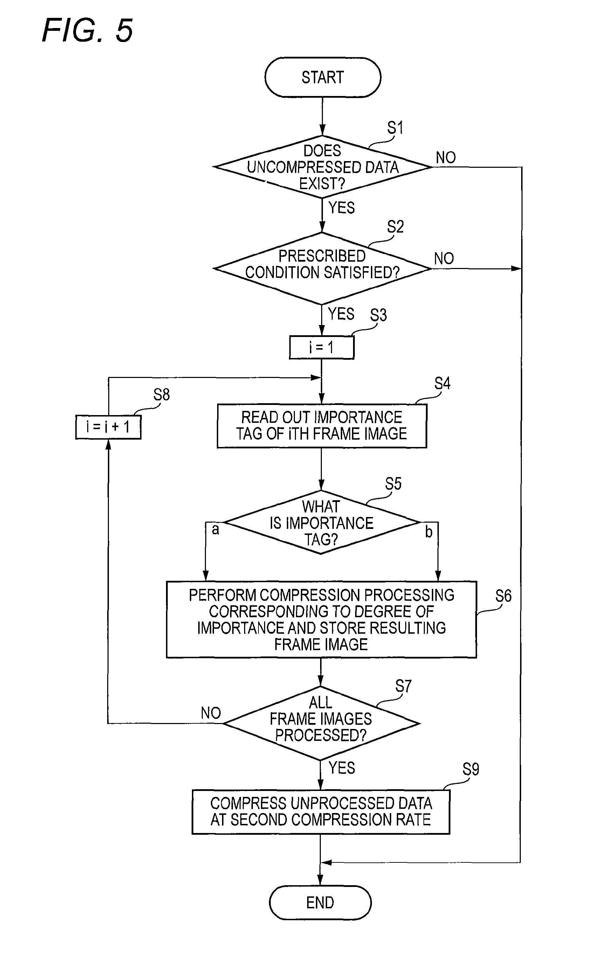

Fig. 5 shows a procedure of compression processing which is performed by a medical image recording program on unprocessed data of moving image data stored in the HDD 88.

The medical image recording program is activated, for example, once each day while the processor unit 14 is free. At step S1, the CPU 82 of the processor unit 14 determines whether or not unprocessed data exists. If no unprocessed data exists, the process is finished. If unprocessed data exists, at step S2 the CPU 82 determines whether or not it satisfies a prescribed condition.

In the embodiment, the prescribed condition may be one such as an elapsed period from a time of recording (during a test or a treatment) or a percentage of use of the HDD 88. More specifically, the prescribed condition may be "a prescribed period (e.g., six months) has elapsed from a test (or treatment)," "the free capacity of the HDD 88 has become smaller than or equal to a prescribed value," or "the ratio of the free capacity of the HDD 88 to its total capacity has become smaller than or equal to a prescribed value."

Another example of the prescribed condition is "prescribed work using the unprocessed data has completed." For example, the CPU 82 determines that the prescribed condition has been satisfied if it detects a push of a report writing completion button of a personal computer of a doctor in charge that is LAN-connected to the processor unit 14 as a result of writing of a report of diagnosis/treatment results using the unprocessed data. The term "prescribed work" is not limited to writing of a report. Still another example of the prescribed condition is "the frequency of access to the unprocessed data has become lower than or equal to a prescribed value."

The process is finished if it is determined at step S2 that the unprocessed data does not satisfy the prescribed condition.

If the unprocessed data satisfies prescribed condition, at step S3 the CPU 82 employs it as a subject of processing and sets a variable i equal to "1." The CPU 82 reads out frame image data having an ith number of the processing subject moving image data from the HDD 88 and transfers it to the compression processing section 85.

At step S4, the compression processing section 85 reads the tag information (importance tag) of the transferred frame image data. At step S5, the compression processing section 85 determines which of "a" and "b" the importance rank is and determines a compression rate corresponding to the thus-found degree of importance by referring to compression rate table data held by the compression rate setting section 86. At step S6, the compression processing section 85 compresses the frame image data at the compression rate corresponding to its importance.

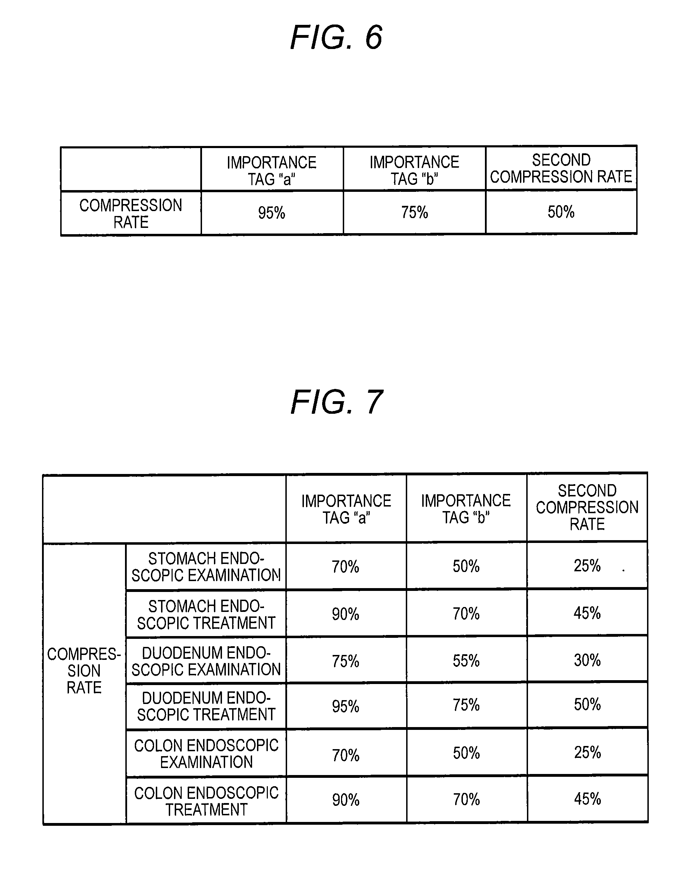

Fig. 6 shows example compression rate table data that is set in the compression rate setting section 86.

In the example of Fig. 6, a compression rate 95% is set for frame image data having the importance tag "a," a compression rate 75% is set for frame image data having the importance tag "b," and a compression rate (second compression rate) 50% is set for the whole of unprocessed data. These compression rate values are just examples. It suffices that the second compression rate for the whole of unprocessed data be smaller than the first compression rate. And the compression rates for frame image data having the importance tag "a" or "b" may be set arbitrarily as long as they are smaller than the first compression rate and larger than the second compression rate.

The compression processing section 85 compresses each frame image data at the corresponding compression rate and stores the compressed frame image data in the HDD 88.

The compression rates may be set in the form of compression schemes instead of numerical values. For example, a reversible compression scheme for a still image such as PNG (portable network graphics) may be set for frame image data having the importance tag "a," a relatively low-compression, irreversible compression scheme for a moving image such as MPEG-2 (Moving Picture Experts Group-2) may be set for frame image data having the importance tag "b," and a relatively high-compression, irreversible compression scheme for a moving image such as MPEG-4 may be set for the whole of unprocessed data.

At step S7, the CPU 82 determines whether or not the current frame image data is the last one. If the current frame image data is the last one, the process is finished. If the current frame image data is not the last one, the variable i is incremented by 1 at step S8 and the process returns to step S4.

As a result of the above steps, the frame image data having the importance tag "a" or "b" are compressed automatically in such a manner that each frame image data is compressed at a compression rate corresponding to its importance. Thus, these frame image data continue to have image quality levels suitable for their uses.

The CPU 82 then sequentially reads out the frame image data of the uncompressed data from the HDD 88 and transfers them to the compression processing section 85.

At step S9, the compression processing section 85 determines a second compression rate for the entire uncompressed data by referring to the compression rate table data held by the compression rate setting section 86, compresses the entire uncompressed data at the thus-determined second compression rate, and records compressed data in the HDD 88, that is, replaces the original unprocessed data with the compressed data.

As a result of the execution of step S9, the unprocessed data is replaced by compressed data that has been compressed at the second compression rate (< (first compression rate)), whereby the free capacity of the HDD 88 is increased.

In the above-described electronic endoscope system 10, in storing image data acquired by the scope 12, frame images that an endoscope operator determines relatively high in importance, such as a release-switch-on frame image and foot-switch-on-period frame images, can be maintained in image quality whereas the entire data amount can be reduced effectively.

In the above-described electronic endoscope system 10, an importance tag "a" is attached to only a release-switch-on frame image (in the example of Fig. 4, #3 frame image). However, taking delay and too early manipulation of the release switch 33 into consideration, importance tags "a" may also be attached to several frame images before and those after the release-switch-on frame image. Likewise, importance tags "b" may also be attached to several frame images before a foot-switch-on frame image (in the example of Fig. 4, #7 frame image) and those after a foot-switch-off frame image (in the example of Fig. 4, #13 frame image).

In the above-described electronic endoscope system 10, the CPU 82 of the processor unit 14 automatically determines a frame image to be extracted as a still image or frame images to be extracted as a partial moving image on the basis of a manipulation signal(s) of the release switch 33 or the foot switch 35. Alternatively, an operator may specify a frame image(s) to be extracted using an input unit such as a keyboard of the processor unit 14 or a keyboard of a personal computer that is LAN-connected to the processor unit 14, while referring to a moving image.

Particular unprocessed data may be left in the HDD 88 by enabling selection as to whether to delete temporarily stored, unprocessed moving image data after its compression described above and allowing an operator to make an instruction to eliminate the particular unprocessed data from the subjects of deletion in advance using the above-mentioned input unit, for example.

Fig. 7 shows another example compression rate table data that is set in the compression rate setting section 86.

Examinations and treatments using electronic endoscope systems are classified into various types such as a stomach endoscopic examination, a duodenum endoscopic examination, a colon endoscopic examination, etc. by the part to be examined and the kind of treatment.

Examination/treatment type information indicating an examination/treatment type is sent from the electronic endoscope system 10 or a LAN-connected intra-hospital system to the processor unit 14 and stored in the HDD 88 so as to be correlated with moving image data acquired by the scope 12.

For example, when an endoscopic resection which is high in risk is conducted, it is preferable to store corresponding frame images of moving image data with a low compression rate (or in an uncompressed state). On the other hand, moving image data of a mere examination may be stored with a high compression rate.

In view of the above, in the compression rate table data shown in Fig. 7, each compression rate is set according to an importance tag that is stored so as to be correlated with frame image data of moving image data and examination/treatment type information that is stored so as to be correlated with the moving image data. In the compression rate table data shown in Fig. 7, each second compression rate for the whole of unprocessed moving image data is set according to examination/treatment type information.

The compression processing section 85 compresses individual frame images and the whole of unprocessed moving image data according to such compression rates, and records compressed frame image data and compressed moving image data in the HDD 88.

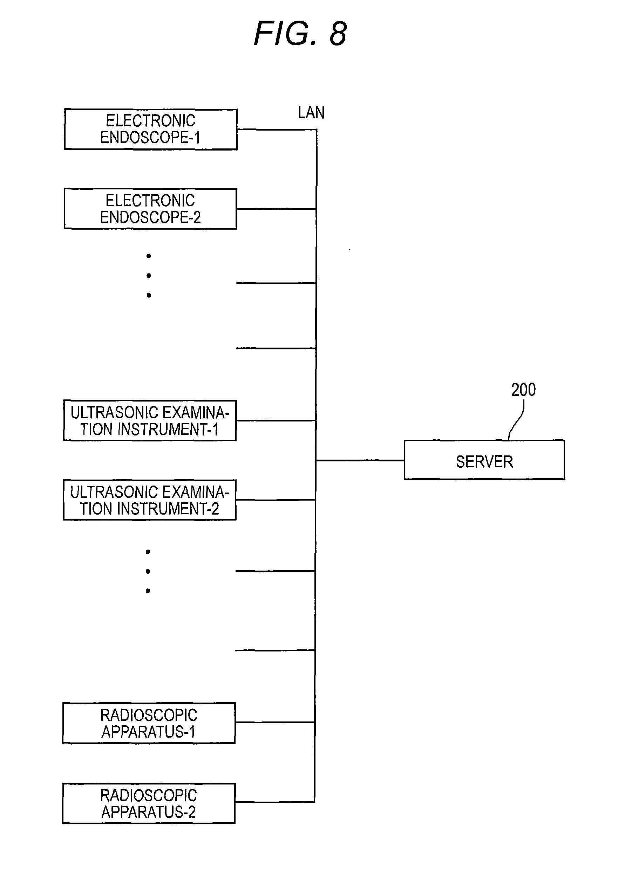

Fig. 8 shows an intra-hospital system which is suitable for a large-scale hospital etc.

The intra-hospital system shown in Fig. 8 is constituted by various modality apparatus and a server 200 to which these modality apparatus are LAN-connected. The types of modality apparatus are an electronic endoscope, an ultrasonic diagnostic instrument, radioscopic apparatus, etc.

Moving image data acquired by each modality apparatus is transferred to the server 200 over the LAN and stored in its large-capacity storage device. Each moving image data is temporarily stored in the large-capacity storage device of the server 200 in such a manner that type information of the modality apparatus is attached to it.

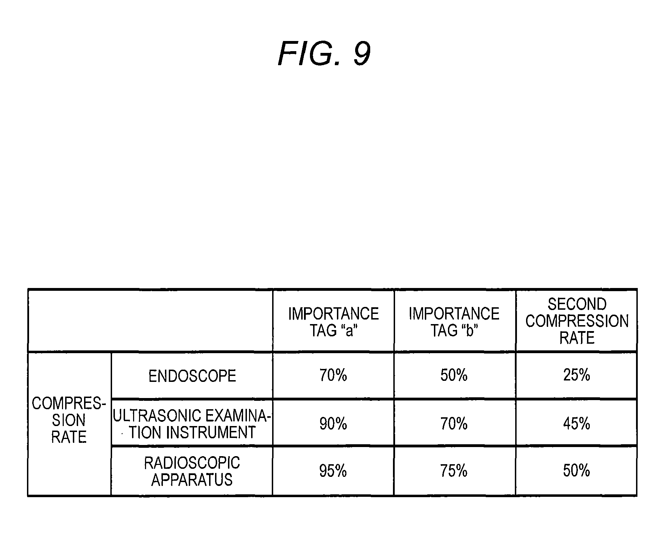

The server 200 is equipped with sections that are similar to the compression processing section 85 and the compression rate setting section 86 of the processor unit 14 of the above-described electronic endoscope system 10. Moving image that is temporarily stored in the large-capacity storage device is compressed according to a procedure that is similar to the above-described moving image data compression procedure of the compression processing section 85 and the compression rate setting section 86. The server 200 performs compression processing according to a modality apparatus type using compression rate table data shown in Fig. 9.

For example, in the case of visible light video of an endoscope, pixel-by-pixel information variations are not important and it has a large amount of information because it is color video. It is therefore desirable to set the compression rate small. In contrast, in the case of ultrasonic video or radiation video, pixel-by-pixel information variations are important in many cases. It is therefore desirable to set the compression rate large.

In view of the above, in the compression rate table data shown in Fig. 9, each compression rate is set according to an importance tag that is stored so as to be correlated with frame image data of moving image data and modality apparatus type information that is stored so as to be correlated with the moving image data. In the compression rate table data shown in Fig. 9, each second compression rate for the whole of unprocessed moving image data is set according to modality apparatus type information.

The compression processing section of the server 200 compresses individual frame images and the whole of unprocessed moving image data according to such compression rates, and records compressed frame image data and compressed moving image data in the large-capacity storage device of the server 200.

In the compression rate table data shown in Fig. 9, each compression rate is set according to an importance tag that is stored so as to be correlated with frame image data of moving image data and modality apparatus type information that is stored so as to be correlated with the moving image data. Alternatively, each compression rate may be set also taking examination/treatment type information into consideration.

In the above embodiment, the degrees of importance of individual frame images constituting moving image data are classified into two ranks (importance tags "a" and "b"). However, it goes without saying that the degrees of importance of frame images may be classified into three ranks, four ranks, or even more ranks.

As described above, the following items are disclosed in the specification:
(1) It is a medical image recording apparatus including: a storage unit that records, at a first compression rate, moving image data acquired by a modality apparatus; a control section that determines degrees of importance of individual frame images constituting the moving image data; and a compression processing section that, if a situation of the moving image data recorded in the storage unit satisfies a prescribed condition, (i) compresses the moving image data at a second compression rate that is smaller than the first compression rate and (ii) compresses each frame image of the moving image data at a compression rate that is smaller than the first compression rate and larger than the second compression rate and that decreases as the degree of importance decreases.
(2) The medical image recording apparatus according to (1), may have a configuration in which the control section sets a degree of importance of part, extracted as a still image or a partial moving image, of frame images constituting the moving image data higher than those of the other frame images.
(3) The medical image recording apparatus according to (1) or (2), may have a configuration in which the control section determines the frame image to be extracted as the still image or the partial moving image on the basis of a manipulation signal or signals of the modality apparatus.
(4) The medical image recording apparatus according to any one of (1) to (3), may further include: an input unit that specifies a frame image, to be extracted as a still image or a partial moving image, of the frame images constituting the moving image data.
(5) The medical image recording apparatus according to any one of (1) to (4), may further include: a compression rate setting section in which compression rates for a frame image of moving image data are set in advance so as to correspond to respective degrees of importance.
(6) The medical image recording apparatus according to any one of (1) to (5), may have a configuration in which the compression processing section compresses each frame image of the moving image data at the compression rate that corresponds to its degree of importance and type of an examination or treatment in which the moving image data is acquired.
(7) The medical image recording apparatus according to any one of (1) to (6), may have a configuration in which the compression processing section compresses each frame image of the moving image data at the compression rate that corresponds to its degree of importance and a type of the modality apparatus.
(8) The medical image recording apparatus according to any one of (1) to (7), may have a configuration in which the compression processing section compresses the moving image data and the individual frame images of the moving image data when a prescribed period has elapsed from the recording of the moving image data in the storage unit.
(9) The medical image recording apparatus according to any one of (1) to (7), may have a configuration in which the compression processing section compresses the moving image data and the individual frame images of the moving image data when a free capacity of the storage unit has become smaller than a prescribed value or a ratio of the free capacity of the storage unit to its total capacity has become smaller than a prescribed value.
(10) The medical image recording apparatus according to any one of (1) to (7), may have a configuration in which the compression processing section compresses the moving image data and the individual frame images of the moving image data if prescribed work using the moving image data has completed.
(11) The medical image recording apparatus according to (10), may have a configuration in which the prescribed work is writing of a report.
(12) The medical image recording apparatus according to any one of (1) to (11), may further include: an input unit that inputs of an instruction to eliminate particular moving image data from subjects of deletion, in which if the moving image data is eliminated form the subjects of deletion, the compression processing section compresses the moving image data at the second compression rate and leaves the moving image data in the storage unit.
(13) It is a medical image recording method including: compressing moving image data acquired by a modality apparatus at a first compression rate and recording resulting moving image data in a storage unit; determining degrees of importance of individual frame images constituting the moving image data recorded in the storage unit; and if a situation of the moving image data recorded in the storage unit satisfies a prescribed condition, (i) compressing the moving image data at a second compression rate that is smaller than the first compression rate and (ii) compressing each frame image at a compression rate that is smaller than the first compression rate and larger than the second compression rate and that decreases as the degree of importance decreases.
(14) It is a medical image recording program which causes a computer to execute: compressing moving image data acquired by a modality apparatus at a first compression rate and recording resulting moving image data in a storage unit; determining degrees of importance of individual frame images constituting the moving image data recorded in the storage unit; and if a situation of the moving image data recorded in the storage unit satisfies a prescribed condition, (i) compressing the moving image data at a second compression rate that is smaller than the first compression rate and (ii) compressing each frame image at a compression rate that is smaller than the first compression rate and larger than the second compression rate and that decreases as the degree of importance decreases.

## Claims

1. A medical image recording apparatus comprising:
a storage unit that records, at a first compression rate, moving image data acquired by a modality apparatus;
a control section that determines degrees of importance of individual frame images constituting the moving image data; and
a compression processing section that, if a situation of the moving image data recorded in the storage unit satisfies a prescribed condition, (i) compresses the moving image data at a second compression rate that is smaller than the first compression rate and (ii) compresses each frame image of the moving image data at a compression rate that is smaller than the first compression rate and larger than the second compression rate and that decreases as the degree of importance decreases.

2. The medical image recording apparatus according to claim 1, wherein
the control section sets a degree of importance of part, extracted as a still image or a partial moving image, of frame images constituting the moving image data higher than those of the other frame images.

3. The medical image recording apparatus according to claim 1 or 2, wherein
the control section determines the frame image to be extracted as the still image or the partial moving image on the basis of a manipulation signal or signals of the modality apparatus.

4. The medical image recording apparatus according to any one of claims 1 to 3, further comprising:
an input unit that specifies a frame image, to be extracted as a still image or a partial moving image, of the frame images constituting the moving image data.

5. The medical image recording apparatus according to any one of claims 1 to 4, further comprising:
a compression rate setting section in which compression rates for a frame image of moving image data are set in advance so as to correspond to respective degrees of importance.

6. The medical image recording apparatus according to any one of claims 1 to 5,
wherein
the compression processing section compresses each frame image of the moving image data at the compression rate that corresponds to its degree of importance and type of an examination or treatment in which the moving image data is acquired.

7. The medical image recording apparatus according to any one of claims 1 to 6,
wherein
the compression processing section compresses each frame image of the moving image data at the compression rate that corresponds to its degree of importance and a type of the modality apparatus.

8. The medical image recording apparatus according to any one of claims 1 to 7,
wherein
the compression processing section compresses the moving image data and the individual frame images of the moving image data when a prescribed period has elapsed from the recording of the moving image data in the storage unit.

9. The medical image recording apparatus according to any one of claims 1 to 7,
wherein
the compression processing section compresses the moving image data and the individual frame images of the moving image data when a free capacity of the storage unit has become smaller than a prescribed value or a ratio of the free capacity of the storage unit to its total capacity has become smaller than a prescribed value.

10. The medical image recording apparatus according to any one of claims 1 to 7,
wherein
the compression processing section compresses the moving image data and the individual frame images of the moving image data if prescribed work using the moving image data has completed.

11. The medical image recording apparatus according to claim 10, wherein
the prescribed work is writing of a report.

12. The medical image recording apparatus according to any one of claims 1 to 11, further comprising:
an input unit that inputs of an instruction to eliminate particular moving image data from subjects of deletion, wherein
if the moving image data is eliminated form the subjects of deletion, the compression processing section compresses the moving image data at the second compression rate and leaves the moving image data in the storage unit.

13. A medical image recording method comprising:
compressing moving image data acquired by a modality apparatus at a first compression rate and recording resulting moving image data in a storage unit;
determining degrees of importance of individual frame images constituting the moving image data recorded in the storage unit; and
if a situation of the moving image data recorded in the storage unit satisfies a prescribed condition, (i) compressing the moving image data at a second compression rate that is smaller than the first compression rate and (ii) compressing each frame image at a compression rate that is smaller than the first compression rate and larger than the second compression rate and that decreases as the degree of importance decreases.

14. A medical image recording program which causes a computer to execute:
compressing moving image data acquired by a modality apparatus at a first compression rate and recording resulting moving image data in a storage unit;
determining degrees of importance of individual frame images constituting the moving image data recorded in the storage unit; and
if a situation of the moving image data recorded in the storage unit satisfies a prescribed condition, (i) compressing the moving image data at a second compression rate that is smaller than the first compression rate and (ii) compressing each frame image at a compression rate that is smaller than the first compression rate and larger than the second compression rate and that decreases as the degree of importance decreases.
